(19) Europäisches Patentamt
European Patent Office
Office européen des brevets

(11) Publication number: 0 228 996 A2

(12) # EUROPEAN PATENT APPLICATION

(21) Application number: 86810593.3

(22) Date of filing: 16.12.86

(51) Int. Cl.$^4$: **A 61 K 31/19** //(A61K31/19,31:12)

(30) Priority: 19.12.85 US 811085

(43) Date of publication of application: 15.07.87 Bulletin 87/29

(84) Designated Contracting States: BE CH DE FR GB IT LI NL SE

(71) Applicant: WARNER-LAMBERT COMPANY
201 Tabor Road
Morris Plains New Jersey 07950 (US)

(72) Inventor: Smerbeck, Richard V.
1811 Hayden Road
Germantown, Tennessee 38138 (US)

Pittz, Eugene P.
7633 Cooper Avenue
Lincoln Nebraska 68516 (US)

(74) Representative: Silbiger, Jakob, Dr.
Münchensteinerstrasse 41 Postfach
CH-4002 Basel (CH)

(54) **Antiflaking agent.**

(57) Medications. medical and cosmetic compositions containing as active agents benzoylperoxide and 2-hydroxy-4-methoxy-benzophenone.

EP 0 228 996 A2

## Description

### ANTIFLAKING AGENT

The present invention relates to a non-steroidal compound useful as an active agent in the treatment of desquamation. This invention also relates to pharmaceutically acceptable compositions containing these agents as well as a method of treatment of desquamation.

Non-steroidal anti-inflammatory compounds are well-known in the art. Examples of such compounds are aspirin. indomethacin. and phenylbutazone. All of these have claims associated with them for the treatment of pain and inflammation in mammals. These compounds are known to cause side effects, gastroenteric disorders and headaches.

U.S. Patent No. 4,145,444 to Hamazaki et al.. discloses various non-carboxylic benzoyl derivatives as anti-inflammatory agents. In particular. those compounds disclosed have the formula:

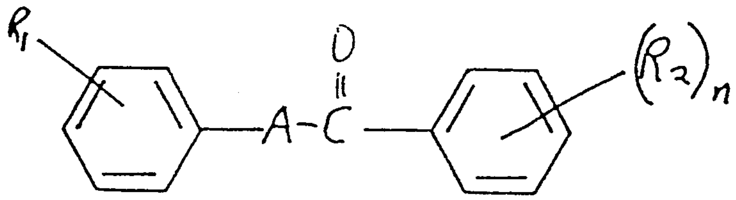

wherein $R_1$ represents hydrogen, halogen, hydroxy, $C_{1-8}$ alkyl or $C_{1-8}$ alkoxy; $R_2$ represents hydrogen, halogen, hydroxy, vinyl, $C_{1-8}$ alkyl or $C_{1-8}$ alkoxy; A represents carbonyl, methylene or a single bond: and n is an integer of l to 4. It is preferred that $R_2$ be n-butyl substituted in the ortho or para position, and $R_1$ be hydrogen or halogen, e.g., compounds having the chemical name 4-n-butylbenzophenone or 4-n-butyl-2′-fluorobenzophenone.

U.S. Patent No. 4,244,970 to Dewhirst discloses a method of treating inflammation and inhibiting prostaglandin synthesis by administering an effective amount of 2-hydroxy-4-methoxybenzophenone and its homologues. This patent discloses those compounds having one hydroxy group located at the ortho position on at least one of the benzene rings.

Desquamation, however, is not synonymous with inflammation. Desquamation is the shedding or scaling of skin of mammals and can be caused by cold dry weather or as the result of contact with certain other factors.

Benzoyl peroxide also known as dibenzoyl-peroxide has been found to be an effective treatment for acne vulgaris (see for example U.S. Patents 4,355,026; 4,318,907 and 4,355,028 issued to Kligman et al.). One of the after effects of the use of benzoyl peroxide is desquamation. This unsightly shedding which results, is cosmetically undesirable.

It has now been found that desquamation resulting from the treatment of benzoyl peroxide can be controlled and minimized by combining benzoyl peroxide with an effective amount of 2-hydroxy 4 methoxy benzophenone.

Dosage forms include medications, pharmaceutical and cosmetic preparations such as topical creams, pastes, ointments, gels, lotions and the like, for direct application to the affected area. Oral dosage forms include, but are not limited to capsules, tablets, solutions, syrups, powders and the like. Rectal, perlingual and parenteral dosage forms are also contemplated.

2-hydroxy,4-methoxybenzophenone may be added directly to commercially available benzoyl peroxide based compositions such as acne preparations at levels ranging from 0.0l to 25% by weight of the preparation or a separate dosage form may be created. Alternatively, dosage in a topical lotion comprising both 2-hydroxy-4-methoxybenzophone and benzoyl peroxide may be added in a pharmaceutically acceptable carrier. Illustrations of useful carriers include ethanol and other lower alkyl alcohols, polyalcohols, mineral oils, vegetable oils, petrolatum, glycerine, nonionic surfactants, water and the like, as well as mixtures of these. As was the case with direct addition to a commercial benzoyl peroxide containing acne treatment.

The two active compounds of the instant invention can be applied together with anti-inflammatory agents, analgesics, thrombus dissolving agents, thrombus inhibiting agents, antibiotics, antihistamines and the like.

The ratio of benzoyl peroxide to 2-hydroxy-4-methylbenzophenone is preferably between 3:l to l:3.

In the case where the active compound is incorporated in a pharmaceutical combination, other common materials such as lubricants, humectants, surfactants, waxes, emulsifiers, thickeners, emollients, preservatives, demulcents, perfumes, coloring additives and the like may be added. These, of course, are not critical to the invention and their amounts can be varied and balanced to meet the desired properties of the overall composition, which is discoverable by routine experimentation by one skilled in the art.

The instant composition may include materials that serve as occlusives in that they hold moisture against the surface of the skin. Suitable occlusive compounds include cetyl alcohol, cetyl palmitate, petrolatum, mineral oil and the like. These materials are generally present in topical compositions, for example, in amounts of about l% to about 25% by weight of the composition and preferably in amounts of about 2% to about l0%.

A variety of materials may be utilized as emulsifiers, including high molecular weight polyethylene glycols,

fatty alcohols such as stearyl alcohol and myristyl alcohol and the like. These materials are generally present in amounts of about 0.1% to about 15% by weight of the composition and preferably in amounts of about 1% to about 10%.

Suitable emollients for use in the instant compositions include fatty acid esters such as cetyl palmitate, diisopropyl adipate, isopropyl isostearate, isostearyl isostearate and mixtures thereof. Generally they are present in topical compositions in amounts of about 0.1% to about 20% by weight of the composition and preferably in amounts of about 1% to about 10%.

Suitable humectants may be any of those well known in the art. Examples of useful humectants include glycerin, propylene glycol, polyethylene glycol, polyhydric alcohols and mixtures thereof. Preferably, glycerin in used. These materials may be incorporated in the inventive compositions in amounts of about 0.1% to about 30% by weight of the composition and preferably in amounts of about 3% to about 20%.

Numerous surfactants, and preferably non-ionic surfactants, may be added for their intended purpose. Among those preferred are polyalkanolamines such as triethanolamine, polyethylene glycol stearate, polyethylene glycol laurate, polyoxyethylene and polyoxypropylene compounds, e.g. as derivatives or sorbitan and fatty alcohol esters, fatty acid esters of polyhydric alcohols and amine oxides; anionic surfactants, such as alkyl carboxylates, acyl lactylates, sulfuric acid esters (e.g. sodium lauryl sulfate), ester-linked sulfonates, and phosphated ethyoxylated alcohols; cationic surfactants, such as monoalkyl and dialkyl quaternary ammonium salts, amidoamides and aminimides. These various surfactants, when compatable, can be added as mixtures to the instant compositions and are generally present in amounts of about 0.01% to about 15% by weight of the composition.

Lubricating agents may be used when desired in the instant compositions. They include silicone oils or fluids such as substituted and unsubstituted polysiloxanes, e.g. dimethyl polysiloxane, also known as dimethicone, is particularly useful when the composition is to be used as a topical preparation. The lubricating agents, when incorporated in a topical composition, are generally present in amounts of about 0.1% to about 30% by weight of the composition and preferably in amounts of about 1% to about 10%. Other lubricating agents well known to the tableting and capsule art may be used when the dosage form is a tablet, pill or capsule. These lubricating agents are primarily to aid in formation of tablets.

Preservatives such as alkyl and aryl parabens and substituted phenols are also useful additives. Examples of the preferred parabens are the methyl, propyl and butyl parabens useful in ranges of 0.1 to about 0.25%.

In a preferred embodiment, a combination of methyl, propyl and butyl paraben may be used in the respective ranges of about 0.1% to about 0.25%, 0.02% to about 0.2% and 0 to about 0.05%. Examples of the useful substituted phenols include chloro-substituted phenoxy phenols, such as 5-chloro-2(2,4-dichloro-phenoxy) phenol, hexacholophene, triclosan and dichlorophene, among others.

Other useful preservatives include mercury derivatives, such as phenylmercuric acetate; quarternaries, such as benzethonium chloride, benzalkonium chlorides and cetyl trimethyl ammonium bromide; acids, such as sorbic acid; and a variety of other preservatives such as Kathon CG, a trademark of Rohm & Haas & Co., Philadelphia, PA 19105 which comprises a mixture of 5-chloro-2-methyl-4-isothiazolin-3-one and 2-methyl-4-isothiazolin-3-one.

Other conventional additives may be utilized, such as fragrance oils, thickeners, emulsifiers and other additives. For example, in the case of a topical lotion, thickeners for viscosity adjustment would include zanthan gum, sodium stearyl sulfate, and materials of that type.

The foregoing recitation of materials is presented for purposes of illustration and not limitation, it being understood that a variety of equivalent materials would all function in the capacities set forth above.

The instant invention also includes a method of treatment for inflammation, pain and related symptoms whereby a mammal is administered a therapeutically effective amount of a composition comprising a compound of the formula

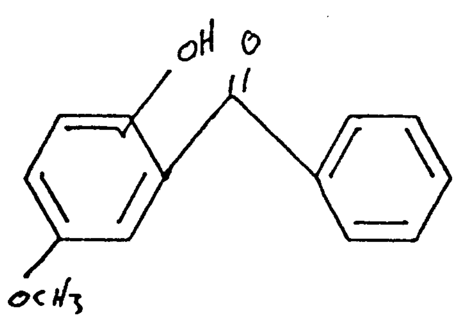

and benzoyl peroxide.

The invention will be further appreciated by the following example which is intended to illustrate an embodiment of the instant invention. All percentages throughout the specification are by weight of the total composition unless otherwise indicated.

The medications, pharmaceutical and cosmetic compositions are produced in a manner known per se.

EXAMPLE

A study using a surfactant accommodated guinea pig was carried out in order to judge the effectiveness of 2-hydroxy-4-methoxybenzophenone as an antiscaling and antiflaking agent in the presence of benzoyl peroxide. The methodology for surfactant treatment consisted of topically applying prototype formulations to respective sites delineated on the abdominal surface of 5 guinea pigs who skin has been made sensitive (e.g., accommodated to a dry, chapped condition) by conditioning with surfactants. The time course of alteration in skin condition due to prototype product application is assessed over an 18-day period by monitoring 11 parameters indicative of the animals' skin condition.

2-hydroxy-4-methoxy benzophenone was added at a level of 6% by weight to Oxy5 to provide the testing solution and set aside for subsequent application. Oxy5 a trademark of Norcliff Thayer, Tuckahoe, NY 10707 for an over-the-counter preparation for acne treatment which contains active Benzoyl peroxide 5% in a carrier including: water, cetyl alcohol, silica, propylene glycol, citric acid, sodium citrate, sodium lauryl sulfate, methyl paraben, and propyl paraben.

Sodium linear alkylbenzene sulfonate (LAS) was obtained from Onyx Chemical Co.. Blue Island, Il 60406, as a light amber liquid containing a minimum of 37% LAS (SURCO 40 SX). This stock solution was diluted to 0.25% LAS with tap water (38-40°C) just prior to the immersion of animals.

Five male albino guinea pigs (700-950 grams) of the Hartley strain were housed singly through the acclimation phase as well as during the course of the study. At the beginning of acclimation (approximately 10 days before the start of the study) the entire abdominal region on each animal was depilated with Neet®, depilating cream (Neet is a trademark of American Home Products Co., New York, NY 10017) and the stomach region divided into 9 sections by permanently outlining regions with black ink delivered from a tattoo gun (model SSMC-1), Spauling & Rogers Mfg. Co., Voorheesville, NJ 12186).

This procedure was also used to affix permanent study numbers to the test animals. Before tattooing, the stomach was treated with 100% ethanol. During and after the tattooing procedures, Carbolated petrolatum was applied to the regions undergoing this procedure.

During the acclimation phase and through the course of the study the animals were fed standard guinea pig diet and given tap water ad libitum. Animal body weights were then taken, and the animals were observed for skin defects and sickness. The room in which the animals were housed was kept on a 12 hour light, 12 hour darkness cycle. The relative humidity and temperature in the test facility was monitored on a 24 hour basis through the course of the study.

During the accommodation phase of the study (days 1-12) the following procedures were carried out:

On a 7-day per week basis and before any other procedures were carried out, the animals' abdominal skin condition was evaluated for flaking. Flaking was evaluated on a visual basis using a Woods' (U.V.A.) light with magnifying glass to enhance visual perception. The scoring system is as follows:

Flaking

0 - No response
1 - Slight response
2 - Moderate scaling
3 - Moderate scaling with some sloughing of epidermis
4 - Severe scaling, sloughing of epidermis, marked cracking
5 - Sloughing of large areas of epidermis, deep cracking with possible hemorrhage

Following the procedure described above, each animal's abdominal region was closely clipped of hair (7 days/week) using standard animals clippers with a No. 40 head.

Next, the five animals were immersed for exactly 30 minutes in 0.25% LAS. This procedure took place daily each morning. During this immersion phase, the animals were placed in perforated, plastic coated steel containers with "loc on" lids. These canisters were then placed in 2 liter beakers containing approximately 1.2 liters of surfactant solution (38 ± 2°C). These beakers were then placed in a water bath maintained at 38 ± 2°C for the 30-minute immersion period.

Immediately after completion of the immersion phase, each animal (in its container) was removed from the beaker and briefly rinsed with tap water that was previously brought to body temperature.

Next, each animal was removed from the canister and placed in an open case which was brought to a surface temperature of approximately 90°F was an infrared light. After 30 minutes of drying the animals were returned to their cages.

On day 13, flakingand TEWL (transepidermal water loss) measurements were taken. TEWL measurements were obtained with the Servo Med Evaporimeter Ep1 (Stockholm, Sweden). The products were then applied to their respective sites.

Approximately 0.3 ml of each product was applied to each site. While wearing rubber gloves, the products were gently massaged into the skin so that each site was thoroughly covered with a thin film of product. Excess product was removed, and care was taken not to cross-contaminate sites. The sites were rotated from animal to animal to avoid positional effects.

Exactly 1 hour after application of products, the animals were immersed in 0.25% LAS for 30 minutes, rinsed, dried and returned to their cages as has been described under Phase 2 procedures. These procedures continued tor 3 days until the next study phase was initiated.

At day 17, the animals' skin condition was evaluated and products applied to their respective sites as

described under Day l3 procedures. However, immersions in LAS was not carried out, thereby allowing the animals' skin condition to regress. The duration of this phase of the study was from day l7 through day 29.

On the day before sacrifice, the animals' skin condition was evaluated as described previously in days l to l2. In addition, both silicone rubber replicas of skin and skin surface biopsies of each site on each animal were taken. Silicone rubber replicas were made by first mixing a predetermined volume of silicone rubber (Dow Corning 3ll0 RTV white silicone rubber) with catalyst (Down Corning RTV Catalyst F) in a ratio of approximately l0Ll. This phase was the applied immediately to each test site on the guinea pigs' stomachs and allowed to set for l5 minutes. Each replica was then removed, identified with a marker pen and stored for further processing into positives. Positives of the skin surface were made with melted polyethylene. These replicas were then processed for SEM analysis by Schick Research and Development.

Skin surface biopsies were performed by first smearing a drop of "Krazy Glue®" (Krazy Glue Inc., Itasca, IL) on the scored surface of an SEM (scanning electron microscope) mount (l8500 Specimen mount from Ernest F. Fullam, Inc., P.O. Box 444, Schenectady. NY l230l) and then placing this mount on a small area of the site (approximately 2 $cm^2$) to be studied. The amount was allowed to become adherent to the site for 5 minutes. Next the amount was gently pulled from the skin surface. A discernable layer of tissue was removed and stored for further processing.

Products were then applied to their respective sites taking care to avoid the areas on each site where surface biopsies were taken.

On the last day of the study, the animals were anesthetized with ether (reagent grade) and full thickness skin biopsies of each test site were taken. These tissues were immediately prepared for histology by fixation in formalin. Histological evaluations were cared out on tissues treated with standard fixing, embedding and Hematoxlylin histological evaluation was performed by Pathology for Toxicology Research, Inc., Tenafly, N.J. The animals were humanely sacrificed and disposed of by CPT.

As noted during the earlier parts of the study, the animals; skin condition was monitored for twenty-four hour post treatment evaluations of flaking, roughness, TEWL (transepidermal water loss) and stratum corneum turnover rate. Each of these parameters will be discussed separately.

Skin flaking was monitored on a daily basis through days l through 29 of the study.

During this phase, flaking scores reached maximum values between days 7 and 8, and subsided to 'steady state' values by day l2. These results are in accord with previous observations [l,2].

Marked differences in flaking responses on sites treated with the combination of this invention when compared with Oxy 5 alone. Duncan's Multiple Range tests were applied to flaking scores on a day to day basis. These results indicate that on days l8, l9, 20, 26 and 27, significant differences in flaking scores on sites treated with various formulations are seen. Generally, it is seen that Oxy 5® causes significantly more flaking while Oxy 5® with 6% W 2-hydroxy-4-methoxybenzophenone causes less flaking. It is also noted that the sites treated with snow white petrolatum (D), snow white petrolatum with 6% W 2-hydroxy-4-methoxybenzophenone, and the untreated site, display less flaking.

The areas under the flaking vs. time curves were statistically evaluated using analysis of variance. The results are shown below:

| | | Site | | |
|---|---|---|---|---|
| Response | p | Oxy 5 | Untreated | Benzophenone |
| Flaking | (<.001) | 40.7 | 33.5 | 31.2 |

It is seen that the product treated sites are split into two classes according to their flaking scores. Of great interest if the fact that addition of 6% W 2-hydroxy-4-methoxybenzophenone to Oxy 5® effected the lowest flaking response compared to Oxy 5® alone which had the highest flaking response.

Cyanoacrylate biopsies and silicone surface replicas taken during Phase 4 of the study were sent to the Schick R&D Group for SEM evaluation. Biopsy and replica samples of each site were evaluated, and a representative area was photographed at 500-520X. The photographs were categorized according to animal and sample type (skin surface biopsy or skin surface replica) and presented to three unbiased individuals for ranking. They ranked the photographs from smoothest skin surface to roughest. A numerical value was then assigned to each ranking (l for smoothest the 9 for roughest). The mean of the rankings for each product was then determined and used as the indicator for surface and deep epidermal effect.

The surface replicas are representative of the animals' surface topology. Evauation of their SEM photographs thus reveal surface alterations caused by treatment with an agent or formulation.

The results are indicated in the Table below:

| Sample | Rank |
|---|---|
| Oxy 5 - Benzophenone | 2.93 |
| Oxy 5 | 4.8 |

## Claims

I. Medication. characterized in that it is composed of benzoyl peroxide and 2-hydroxy-4-methoxybenzophenone.

2. A pharmaceutical composition, characterized, in that it contains as active principles benzoyl peroxide and 2-hydroxy-4-methoxybenzophenone.

3. A cosmetic composition, characterized in that it contains benzoyl peroxide and 2-hydroxy-4-methoxybenzophenone.

4. The medication, pharmaceutical composition or cosmetic composition according to claim I - 3 wherein 2-hydroxy-4-methoxybenzophenone is present between 0.0I and 25% by weight of the combination.

5. The medication, pharmaceutical composition or cosmetic composition according to claim 4, wherein 2-hydroxy-4-methoxybenzophenone is present at a level of from 3 to I0% by weight of the combination.

6. The medication, pharmaceutical composition or cosmetic composition according to the claims I - 5 wherein the ratio of benzoyl peroxide to 2-hydroxy-4-methylbenzophenone is between 3:I and I:3.

7. A medication, pharmaceutical composition or cosmetic composition, according to the claims I - 6, for the treatment of desquamation in mammals.

8. The method of use of a combination containing benzoylperoxide and 2-hydroxy-4-methoxybenzophenone for the manufacture of a medication, a pharmaceutical composition or a cosmetic composition as claimed in the claims I to 7.

9. A method of use according to claim 8 for the manufacture of a medication, a pharmaceutical composition or a cosmetic composition for the treatment of desquamation in mammals.